# EUROPEAN PATENT SPECIFICATION

(11) **EP 3 497 617 B1**
(45) Date of publication and mention of the grant of the patent: **23.09.2026**
(21) Application number: 17752467.5
(22) Date of filing: 10.08.2017
(51) Int. Cl.: G06V 10/145, G06V 40/12, G06V 40/13

(54) **SKINPRINT ANALYSIS METHOD AND APPARATUS**
VERFAHREN UND VORRICHTUNG ZUR ANALYSE EINES HAUTABDRUCKS
PROCÉDÉ ET APPAREIL D'ANALYSE D'EMPREINTE CUTANÉE

(30) Priority: 11.08.2016 GB 201613819
(43) Date of publication of application: 19.06.2019
(73) Proprietor: Intelligent Fingerprinting Limited, Cambridgeshire CB24 9NG (GB)
(72) Inventor: GORDON, Benjamin, Cambridge Cambridgeshire CB24 9NG (GB); WILSON, Paul, Cambridge Cambridgeshire CB24 9NG (GB)
(74) Representative: Boult Wade Tennant LLP
(86) International application number: PCT/GB2017/052365
(87) International publication number: WO 2018/029482

(56) References cited:
- US-A1- 2013 285 977
- US-A1- 2015 205 992
- US-B1- 6 292 576

## Description

### Technical Field

The disclosure relates to analysis of a skinprint, such as a fingerprint. The analysis may confirm the presence of a skinprint and may also provide an indication of the quality and/or quantity of the skinprint. The analysis may also confirm the identity of the skinprint.

### Background

Skinprints comprise eccrine sweat and may contain other constituents that may form a target for a diagnostic test. The applicant has developed a range of techniques for detecting the presence of one or more analytes in skinprints.

Characteristics of skinprints may vary substantially, for example in terms of area of skinprint and quantity of print substances present in the skinprint. These and other characteristics may contribute to a measure of skinprint quality/quantity. Skinprint quality/quantity may considerably affect the ease or otherwise of analysing the skinprint both for identification purposes and also for the purpose of detecting one or more analytes in the skinprint, especially when seeking quantitative analysis of one or more analytes.

By way of example, a good quality skinprint, on which the detection of one or more analytes may be most straightforward, may be one that is provided by a user depositing a firm impression of unwashed skin on a surface. By contrast, a reduced quality skinprint may be provided by a user who has recently washed the relevant area of skin and/or who provides only a minimal force when depositing the impression on a surface.

In some circumstances, for example, it may be that a user deliberately washes their hands in anticipation of being asked to provide a fingerprint and also uses only a minimal force when leaving the fingerprint.

It may be beneficial to analyse a skinprint for a measure of quality/quantity for a number of reasons. Such reasons may include (but are not necessarily limited to): confirming that a skinprint is present at all; confirming (where a skinprint is present) that the skinprint is of sufficient quality/quantity to facilitate a meaningful analysis of analytes; determining (where a skinprint is present) whether quality/quantity is sufficient for a quantitative analysis of analytes to be performed.

In addition, providing a straightforward quality check may avoid cost and inefficiency associated with analyte testing on a sample that is sub-standard or even absent altogether.

A quality check may be undertaken before or after collecting a skinprint for diagnostic analysis as well as being an integral part of a diagnostic analysis of materials such as metabolites in the skinprint.

It is known to use a quartz crystal microbalance to measure small mass increments. This technique does not lend itself well to robust in-the-field determination of fingerprint or skinprint mass measurement. Furthermore, while skinprint quantity may correspond with mass, the relationship between skinprint quality and mass may be more complex. The applicant has identified a need for a rugged, reliable system with low cost consumables for the purpose of analysing skinprint quality/quantity.

US 2015/205992 discloses a method for determining presence of skin in situ rather than residue of constituents of a skinprint that is residual following removal of the skin.

### Summary of the disclosure

Against this background, there is provided a method of determining presence of residue of constituents of a skinprint using an apparatus comprising: a primary electromagnetic radiation source; an electromagnetic radiation detector; and a translucent waveguide comprising a first surface providing a waveguide interface coincident with a skinprint receiving region;
the method comprising:
transmitting primary electromagnetic radiation from the primary electromagnetic radiation source towards the waveguide interface at an angle of incidence relative to and on a first side of a normal line that is perpendicular to the waveguide interface, such that:
   (a) where the waveguide interface interfaces directly with ambient air, the primary electromagnetic radiation incident on the waveguide interface reflects in the waveguide interface at an angle of reflection relative to and on a second side of the normal line opposite the first side; and
   (b) where residue of constituents of a skinprint is present on the skinprint receiving region such that the waveguide interface interfaces with the residue of constituents of the skinprint and the residue of constituents of the skinprint interfaces with ambient air, at least a portion of the primary electromagnetic radiation incident on the waveguide interface is coupled into or out of the translucent waveguide via the residue of constituents of the skinprint; and
using the electromagnetic radiation detector to determine an amount of primary electromagnetic radiation transmitted through the waveguide interface or reflected by the waveguide interface; and
where residue of constituents of the skinprint is present, calculating a quality metric for the residue of constituents of the skinprint based at least in part on an amount of primary electromagnetic radiation detected by the electromagnetic radiation detector to provide a value indicative of strength and/or extent of residue of constituents of the skinprint on the skinprint receiving region.

In this way, residue of constituents of a skinprint may be used to couple electromagnetic radiation into or out of a translucent waveguide. The extent of the coupled electromagnetic radiation is detected or at least inferred and thereby provides an indication of the quality/quantity of the impression of the residue of constituents of the skinprint.

Also disclosed is an apparatus for determining presence of residue of constituents of a skinprint, the apparatus comprising: a primary electromagnetic radiation source; an electromagnetic radiation detector; and a translucent waveguide comprising a first surface providing a waveguide interface coincident with a skinprint receiving region;
wherein the apparatus is configured to:
transmit primary electromagnetic radiation from the primary electromagnetic radiation source towards the waveguide interface at an angle of incidence relative to and on a first side of a normal line that is perpendicular to the waveguide interface, such that:
   (a) where the waveguide interface interfaces directly with ambient air, the primary electromagnetic radiation incident on the waveguide interface reflects in the waveguide interface at an angle of reflection relative to and on a second side of the normal line opposite the first side; and
   (b) where residue of constituents a skinprint is present on the skinprint receiving region such that the waveguide interface interfaces with the residue of constituents of the skinprint and the residue of constituents of the skinprint interfaces with ambient air, at least a portion of the primary electromagnetic radiation incident on the waveguide interface is coupled into or out of the translucent waveguide via the residue of constituents of the skinprint;
use the electromagnetic radiation detector to determine an amount of primary electromagnetic radiation transmitted through the waveguide interface or reflected by the waveguide interface; and
where residue of constituents of the skinprint is present, to calculate a quality metric for the residue of constituents of the skinprint based at least in part on an amount of primary electromagnetic radiation detected by the electromagnetic radiation detector to provide a value indicative of strength and/or extent of residue of constituents of the skinprint on the skinprint receiving region.

Embodiments of the disclosure will now be described, by way of example only, with reference to the accompanying drawing in which:

### Brief description of the drawings

Figure 1a provides a schematic representation of a first embodiment of the disclosure showing behaviour of electromagnetic radiation in the event that an impression of residue of constituents of a skinprint is present;
Figure 1b provides a schematic representation of the first embodiment of the disclosure showing behaviour of electromagnetic radiation in the event that no impression of residue of constituents of a skinprint is present;
Figure 2a provides a schematic representation of a second embodiment of the disclosure showing behaviour of electromagnetic radiation in the event that an impression of a deposited skinprint is present;
Figure 2b provides a schematic representation of the second embodiment of the disclosure showing behaviour of electromagnetic radiation in the event that no impression of a deposited skinprint is present;
Figure 3a provides a schematic representation of a third embodiment of the disclosure showing behaviour of electromagnetic radiation in the event that an impression of a deposited skinprint is present;
Figure 3b provides a schematic representation of the third embodiment of the disclosure showing behaviour of electromagnetic radiation in the event that no impression of a deposited skinprint is present;
Figure 4a provides a schematic representation of a fourth embodiment of the disclosure showing behaviour of electromagnetic radiation in the event that an impression of a deposited skinprint is present;
Figure 4b provides a schematic representation of the fourth embodiment of the disclosure showing behaviour of electromagnetic radiation in the event that no impression of a deposited skinprint is present;
Figure 5a provides a schematic representation of a fifth embodiment of the disclosure showing behaviour of electromagnetic radiation in the event that an impression of a deposited skinprint is present;
Figure 5b provides a schematic representation of the fifth embodiment of the disclosure showing behaviour of electromagnetic radiation in the event that no impression of a deposited skinprint is present;
Figure 6a provides a schematic representation of a sixth embodiment of the disclosure showing behaviour of electromagnetic radiation in the event that an impression of a deposited skinprint is present;
Figure 6b provides a schematic representation of the sixth embodiment of the disclosure showing behaviour of electromagnetic radiation in the event that no impression of a deposited skinprint is present;
Figure 7a provides a schematic representation of a seventh embodiment of the disclosure showing behaviour of electromagnetic radiation in the event that an impression of a deposited skinprint is present;
Figure 7b provides a schematic representation of the seventh embodiment of the disclosure showing behaviour of electromagnetic radiation in the event that no impression of a deposited skinprint is present;
Figure 8a provides a schematic representation of an eighth embodiment of the disclosure showing behaviour of electromagnetic radiation in the event that an impression of a deposited skinprint is present; and
Figure 8b provides a schematic representation of the eighth embodiment of the disclosure showing behaviour of electromagnetic radiation in the event that no impression of a deposited skinprint is present.

### Detailed description

The disclosure relates to a method and apparatus for determining presence of an impression of a deposited skinprint 30. A wide range of alternative implementations is envisaged. The following detailed description relates to a subset of embodiments that fall within the scope of the appended claims. Where this specification uses the term skinprint, this is shorthand for an impression of a deposited skinprint, such as an impression of a deposited fingerprint.

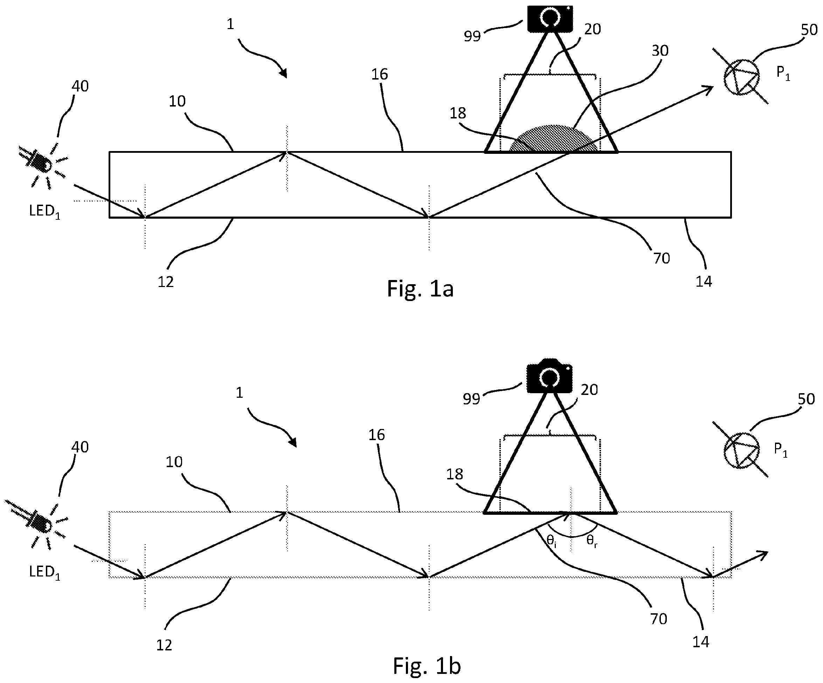

Figure 1a and 1b show a schematic representation of a first embodiment 1 of the disclosure. Figure 1a shows behaviour of electromagnetic radiation in the first embodiment 1 where a skinprint 30 is present while Figure 1b shows behaviour of electromagnetic radiation in the first embodiment where no skinprint is present. The first embodiment 1 comprises an LED 40, a photodiode 50 and a translucent waveguide 10 between the LED 40 and the photodiode 50 configured to output a photodiode signal indicative of electromagnetic radiation detected by the photodiode 50.

The translucent waveguide 10 comprises a first end 12 and a second end 14. The LED 40 is optically coupled to the translucent waveguide 10 towards the first end 12.

The second end 14 comprises a fingerprint receiving region 20 on a first surface 16 of the translucent waveguide 10. The fingerprint receiving region 20 may be identified on the first surface 16 by virtue of one or more visible indications on or surrounding the fingerprint receiving region 20. Alternatively, the fingerprint receiving region 20 may be identified by a window bounded by a frame that obscures parts of the first surface 16 that do not form part of the fingerprint receiving region 20. The fingerprint receiving region 20 may be identified by other means.

A surface of the translucent waveguide 10 in the vicinity of the fingerprint receiving region 20 may serve as a waveguide interface 18 through which electromagnetic radiation may be transmitted or in which electromagnetic radiation may be reflected, dependent upon circumstances. The waveguide interface 18 may or may not be different in surface properties when compared to a surface of the translucent waveguide 10 that surrounds the waveguide interface 18.

The photodiode 50 is located so as to detect electromagnetic radiation that is transmitted out of the translucent waveguide 10 via the waveguide interface 18.

The LED 40 is optically coupled to the translucent waveguide 10 towards the first end 12 such that electromagnetic radiation 70 emitted by the LED 40 enters into the translucent waveguide 10 at an angle such that the electromagnetic radiation 70 is retained within the translucent waveguide by total internal reflection. Optical coupling of the LED 40 to the translucent waveguide 10 may take any appropriate form. At the point of entry of the electromagnetic radiation 70 into the translucent waveguide 10, some refraction of the electromagnetic radiation 70 may take place. (For the sake of clarity, this refraction is not shown in the Figures.) In particular, electromagnetic radiation 70 that is incident upon an end surface of the translucent waveguide 10 at an angle of incidence is transmitted into the translucent waveguide 10 with a small change in direction away from a normal line (which is shown in the Figure) that is perpendicular to the surface through which the electromagnetic radiation 70 enters the translucent waveguide 10. The extent of the refraction that takes place depends upon the ratio between the index of refraction of the translucent waveguide 10 and the index of refraction of the material through which the electromagnetic radiation 70 travels immediately prior to reaching the point of entry. Where the material immediately prior to the electromagnetic radiation 70 reaching the point of entry is ambient air, the ratio is likely to be higher (and so the extent of the refraction is likely to be greater) than if the material immediately prior to the electromagnetic radiation 70 reaching the point of entry is, for example, a translucent encapsulation material of an LED package. Accordingly, the nature and extent of any refraction will depend upon how the electromagnetic radiation 70 is coupled from the electromagnetic radiation source 40 into the translucent waveguide 10.

Subsequently, as the electromagnetic radiation 70 travelling within the translucent waveguide 10 reaches the edges of the translucent waveguide 10, it arrives at an angle of incidence that is such as to cause the electromagnetic radiation 70 to reflect at the perimeter of the translucent waveguide 10 as a result of total internal reflection rather than to be transmitted out of the translucent waveguide 10. This pattern of total internal reflection is reproduced along the translucent waveguide 10 and by this mechanism the electromagnetic radiation 70 propagates along and within the translucent waveguide 10.

While in Figure 1a a skinprint 30 is shown in situ on the skinprint receiving region 20, in Figure 1b no skinprint is present on the skinprint receiving region 20. A comparison between Figures 1a and 1b illustrates how behaviour of electromagnetic radiation 70 is influenced by the presence or absence of a skinprint 30 on the skinprint receiving region.

In the case that no skinprint is present on the skinprint receiving region, as is evident from Figure 1b, a further total internal reflection occurs at the location of the skinprint receiving region 20 such that the electromagnetic radiation 70 continues to propagate along the translucent waveguide 10. When the electromagnetic radiation 70 reaches the end of the translucent waveguide 10, it arrives at an angle such that it passes through the end of the translucent waveguide 10, albeit undergoing some refraction (again for the sake of clarity not shown in Figure 1b) and thereby exits the translucent waveguide 10.

By contrast, as can be seen from Figure 1a, in the case that a skinprint 30 is present on the skinprint receiving region 20, at least a portion of the electromagnetic radiation 70 that arrives at the skinprint receiving region 20 is transmitted out of the translucent waveguide 10 at the waveguide interface 18 by virtue of the presence of the skinprint 30. This is because the waveguide interface 18 is (at least partially) covered by residue of the constituents of the skinprint, hereafter for brevity referred to simply as the skinprint 30. Therefore, instead of the waveguide interface 18 interfacing directly with ambient conditions (such as ambient air) wherein the difference in refractive indices between the translucent waveguide 10 and ambient would be such as to result in total internal reflection, the waveguide interface 18 interfaces directly with the skinprint 30. The ratio of refractive indices between that for the translucent waveguide 10 and that for the skinprint 30 is such that at least some of the electromagnetic radiation 70 is transmitted through the waveguide interface 18 and into the skinprint. When the electromagnetic radiation 70 reaches the surface of the skinprint (opposite the translucent waveguide 10) a combination of the ratio of refractive indices between that for the skinprint 30 and that for the ambient together with the angle of incidence of the electromagnetic radiation 70 at the interface results in at least some of the electromagnetic radiation 70 being transmitted out of the skinprint 30.

In the embodiment of Figures 1a and 1b, electromagnetic radiation 70 that is transmitted via the waveguide interface 18 and out of the skinprint 30 is received at the photodiode 50. In very general terms, the greater the (influence of) the skinprint, the more electromagnetic radiation 70 is received by the photodiode 50. Accordingly, there is a relationship between the quality and/or extent of skinprint 30 on the skinprint receiving region 20 and the amount of electromagnetic radiation 70 detected by the photodiode 50. Where no skinprint is present, little or no electromagnetic radiation 70 will be detected by the photodiode 50 because it remains within the translucent waveguide 10. Where a well-defined, strong skinprint is present, a significant proportion of the electromagnetic radiation 70 will be coupled out of the waveguide interface and will reach the photodiode 50.

It should be noted that Figures 1a and 1b (as well as the corresponding Figures relating to other embodiments) are highly schematic. As the skilled person would readily understand, the analysis is not binary. That is to say, it is not the case that in the event of a skinprint 30 being present all electromagnetic radiation 70 will transmit out of the translucent waveguide 10 via the waveguide interface 18. Similarly, it is not the case that in the event of no skinprint is present, no electromagnetic radiation 70 will transmit out of the translucent waveguide via the waveguide interface 18. In reality, some electromagnetic radiation 70 will transmit out of the translucent waveguide when no skinprint 30 is present. Conversely, when a skinprint 30 is present some electromagnetic radiation 70 will remain in the translucent waveguide.

Furthermore, it should be noted that the electromagnetic radiation 70 will not all travel in exactly the directions indicated by the arrows in Figures 1a and 1b. In short, Figures 1a and 1b are schematic and are intended to illustrate the principles.

In the Figures, the schematic representation of a skinprint 30 (where present) is such as to suggest that it is manifested as a single dome-shaped form on the skinprint receiving region 20. It is emphasised that this representation is highly schematic. Again as the skilled person readily appreciates, the form of skinprints varies significantly depending upon many factors including the amount of eccrine sweat on the surface of the skin when printed and the force with which a user places the skin against the skinprint receiving region 20 when providing a skinprint. In reality, the skinprint is likely to comprise a number of peaks and troughs, all of which may influence the behaviour of electromagnetic radiation incident upon it in a variety of ways.

As can be seen from Figures 1a and 1b, the first embodiment may further comprise optical imaging capability, as illustrated schematically by a camera icon 99. The optical imaging capability may be employed to provide an optical image of the skinprint that might be compared with a database of skinprint images, so as to confirm identity of a skinprint. The optical image functionality is equally applicable to any of the other embodiments disclosed herein but, for the sake of clarity, it is not illustrated other than in Figures 1a and 1b.

The applicant has developed various techniques for chemical analysis of skinprints. In order to determine that the chemical analysis is feasible for a given skinprint, it is helpful to have an indication that there is sufficient material present in a skinprint in order to apply a particular chemical test and, in particular, to quantify results of the chemical analysis relative to a mass or volume of the skinprint under test. The techniques described herein provide an indication of the amount of skinprint (hereinafter referred to skinprint quality) that has been deposited on the skinprint receiving region. Where skinprint quality is high, the influence of the skinprint on the behaviour of electromagnetic radiation will be higher than when the skinprint quality is low which will in turn be higher than when there is no skinprint present.

While the techniques described herein may be useful for providing a binary output that simply indicates whether a skinprint is of sufficient quality for a chemical analysis to be performed (by exceeding a fingerprint quality threshold), for skinprints that meet this threshold it may also be desirable to provide a more granular quantitative output. This may in turn be used to provide an indication of a quantum of a particular chemical constituent that may be expected. For example, a high quality skinprint may be expected to contain more of a particular chemical than a lower (but still adequate) quality skinprint. Accordingly, if the subsequent chemical analysis is intended not only to detect for presence of a chemical but also for an indication of concentration of that chemical, a quantitative analysis of the quality of the skinprint may be used in this determination.

The apparatus of the first embodiment may comprise controller circuitry configured to receive the photodiode signal and process that signal in order to determine whether a skinprint quality threshold is met. It may also be configured to determine a metric for quality of the skinprint. The controller may, for example, be configured to receive a first (reference) photodiode signal prior to a user providing a skinprint on the skinprint receiving region and to receive a second photodiode signal once a skinprint has been provided on the skinprint receiving region and to compare the first and second signals. It may also be configured to make a comparison with a reference value indicative of a theoretical maximum that would be achieved in the event of a maximum quality skinprint. By appropriate processing of the first and second signals relative to the reference value a skinprint quality value may be calculated for a particular skinprint and output via a display or as data transmitted for onward processing and/or storage. Alternatively or in addition, it may be that the result is simply compared to a threshold to determine if it meets a predetermined criterion or criteria for a meaningful analysis and a simple binary output may be provided such as a red/green indication (in the manner of traffic lights).

The skilled person will recognise that there are a large number of options applicable to the first embodiment of the disclosure for processing data such as photodiode signals as well as data relating to the electromagnetic radiation emitted by the LED in order to calculate a skinprint quality value. Moreover, in some of the subsequent embodiments that include additional features and functionality, there may be further inputs for calculation of the skinprint quality value. In some or all of these embodiments, there may be a calibration technique as a precursor to performing the analysis that results in the skinprint quality value.

It may be that where a quantitative skinprint quality value is provided, this provides an input to an algorithm related to chemical analysis of the skinprint thereby providing a reference for an amount of chemical that might be expected in a skinprint of that particular quality value. In addition or instead it may be that the skinprint quality value is output to a chemical analysis process simply to confirm that the skinprint is of sufficient quality/quantity to be appropriate for chemical analysis. In this way, it may be possible to avoid the time and expense associated with performing chemical analysis on a skinprint in which there is no confidence that a meaningful chemical analysis can be performed because the quality/quantity of the skinprint is insufficient.

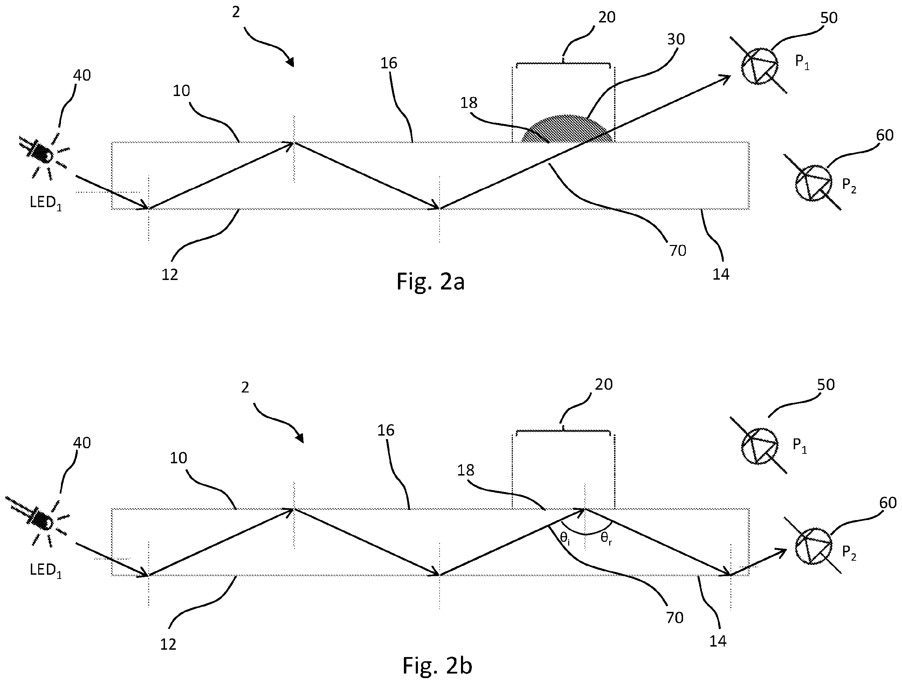

Figures 2a and 2b show a second embodiment 2 of the disclosure. The second embodiment 2 of the disclosure differs from the first embodiment in that a second photodiode 60 is provided in addition to the first photodiode 50. The second photodiode 60 is intended to detect electromagnetic radiation 70 that is not transmitted through the waveguide interface and is instead propagated by total internal reflection throughout the translucent waveguide 10. By providing two photodiodes and obtaining a signal from each indicative of an amount of electromagnetic radiation detected by each, the signals from each of the first and second photodiodes 50, 60 can be compared as part of a calculation to determine a skinprint quality value.

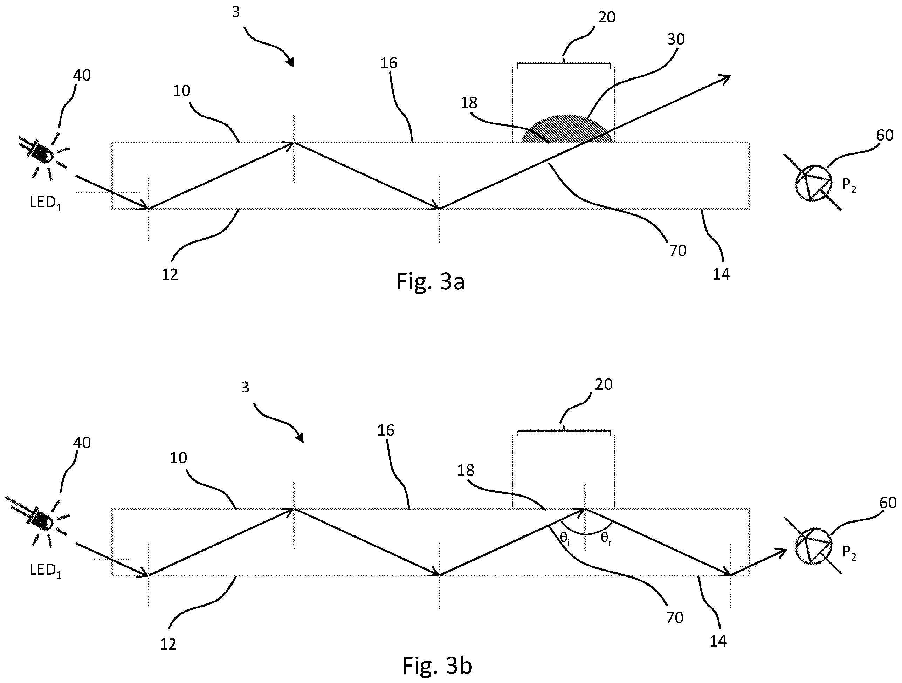

Figures 3a and 3b show a third embodiment 3 of the disclosure. The third embodiment 3 of the disclosure differs from the first and second embodiments 1, 2 in that only the second photodiode 60 (and not the first photodiode) is provided. In this way, the photodiode 60 only detects electromagnetic radiation 70 that is not transmitted through the waveguide interface and is instead propagated by total internal reflection throughout the translucent waveguide 10.

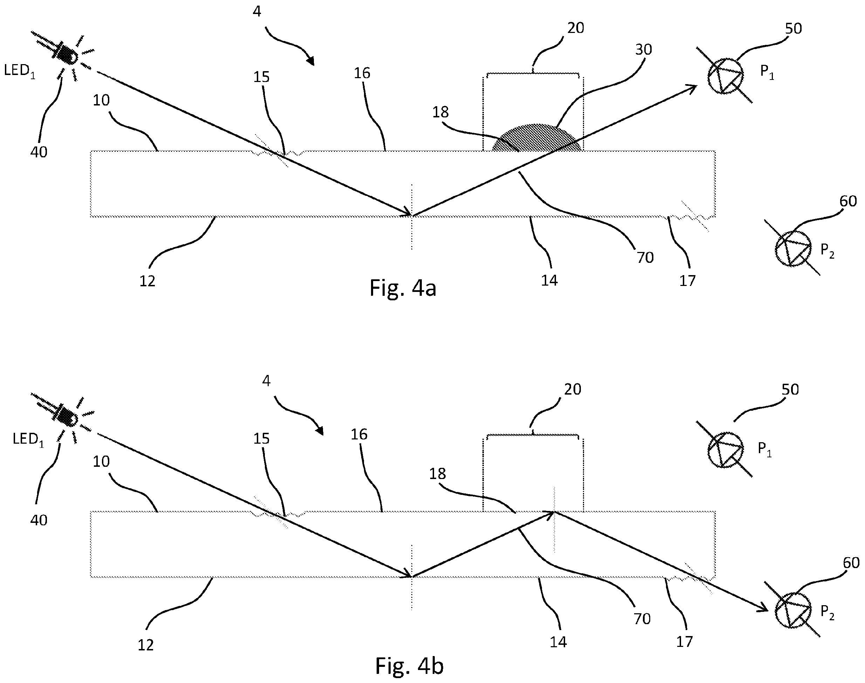

Figures 4a and 4b show a fourth embodiment 4 of the disclosure. The fourth embodiment 4 of the disclosure differs from the second embodiment 2 in that electromagnetic radiation 70 is transmitted (coupled) into the translucent waveguide 10 via a first grating coupler 15 and in that electromagnetic radiation 70 that is not transmitted out of the waveguide interface 18 and continues to propagate through the translucent waveguide 10 by total internal reflection is transmitted (coupled) out of the translucent waveguide 10 via a second grating coupler 17. The first grating coupler 15 may comprise a roughened portion of a surface of the translucent waveguide 10 through which electromagnetic radiation may pass into the translucent waveguide 10. This may provide flexibility regarding location of the LED 40 relative to the translucent waveguide 10. This may be particularly appropriate when providing the apparatus in a compact portable package. The second grating coupler 17 may comprise a roughened portion of a surface of the translucent waveguide 10 through which electromagnetic radiation may pass out of the translucent waveguide 10. This may provide flexibility regarding location of the second photodiode 60 relative to the translucent waveguide 10. Again, this may be particularly appropriate when providing the apparatus in a compact portable package.

As the skilled person would readily appreciate, alternative embodiments (not illustrated) may involve only one of the two grating couplers 15, 17. For example, an alternative embodiment may include a first grating coupler 15 in the absence of a second grating coupler 17. In such an embodiment electromagnetic radiation 70 that is not transmitted out of the waveguide interface 18 and continues to propagate through the translucent waveguide 10 by total internal reflection may be transmitted (coupled) out of the translucent waveguide 10 in the same manner as in the second and third embodiments 2, 3. Similarly, a further alternative embodiment may include a second grating coupler 17 in the absence of a first grating coupler 15. In such an embodiment, electromagnetic radiation 70 may be coupled into the translucent waveguide 10 in the same manner as for the first, second and third embodiments 1, 2, 3.

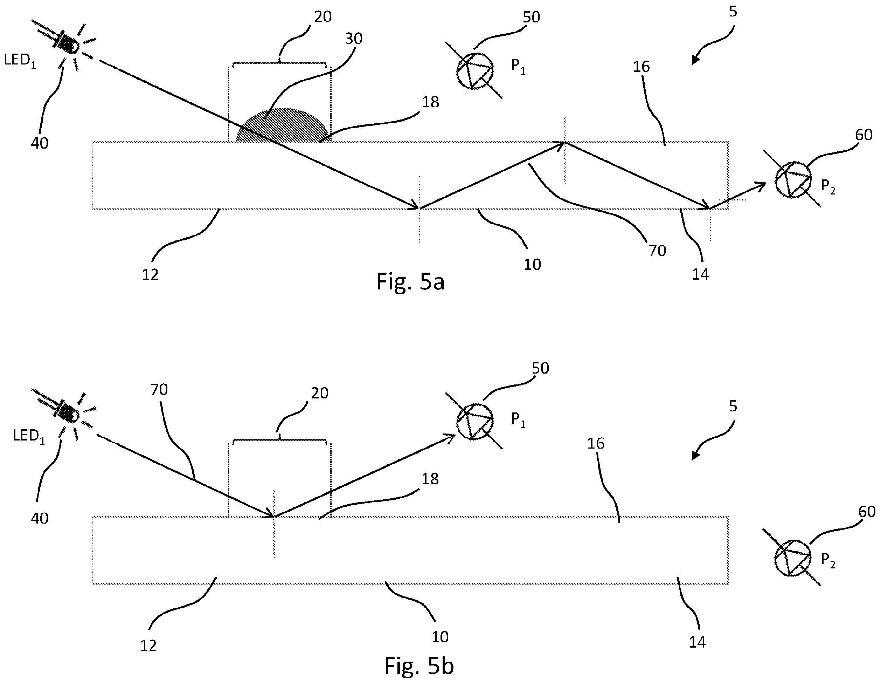

Figures 5a and 5b show a fifth embodiment 5 of the disclosure. The fifth embodiment 5 differs from the first to fourth embodiments 1, 2, 3, 4 in that the direction of potential transmission through the waveguide interface 18 (in the presence of a skinprint) is into the translucent waveguide 10 rather than out of the translucent waveguide 10. Accordingly, the electromagnetic radiation source 40 is located such that electromagnetic radiation 70 reaches the waveguide interface 18 from the exterior of the translucent waveguide 10 towards the first end 12 of the translucent waveguide 10. In addition, the fingerprint receiving region 20 is located on the first surface 16 of the translucent waveguide 10 also towards the first end 12 of the translucent waveguide 10. In the event that a skinprint is present, electromagnetic radiation 70 is transmitted through the waveguide interface 18 and into the translucent waveguide 10 for onward propagation towards the second end 14 of the translucent waveguide 14 through total internal reflection as shown schematically in Figure 5a. In the event that no skinprint is present, electromagnetic radiation simply reflects off the waveguide interface 18 and thereby never enters the translucent waveguide 10 as shown in Figure 5b. In the illustration, electromagnetic radiation that is reflected in the waveguide interface 18 is detectable by a first photodiode 50 and electromagnetic radiation that is transmitted through the waveguide interface 18 via a skinprint is detectable by a second photodiode 60. However, in common with the differences between the first, second and third embodiments 1, 2, 3, it may be appropriate to have only one rather than both of the photodiodes.

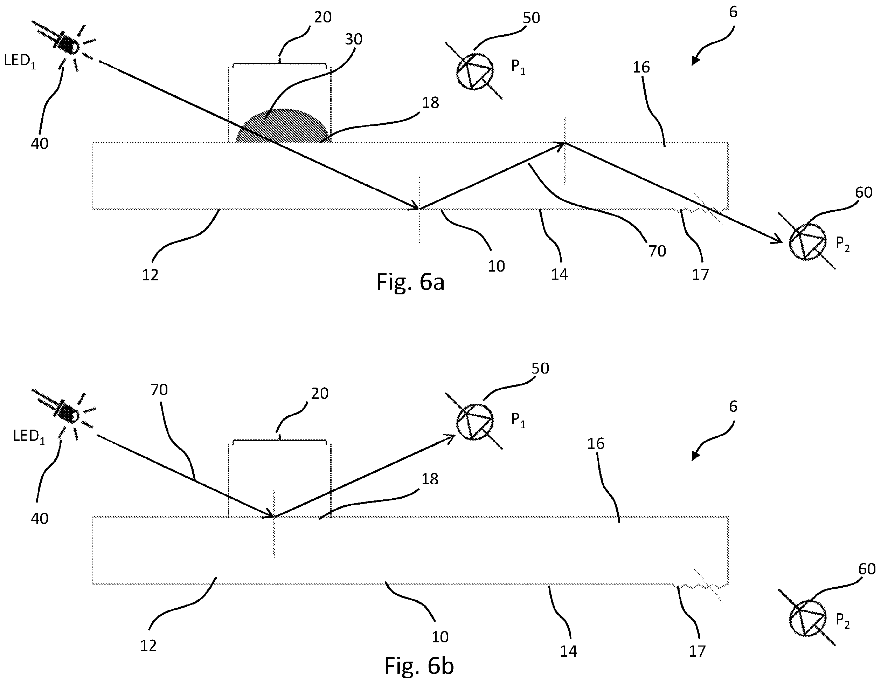

Figures 6a and 6b show a sixth embodiment 6 of the disclosure. The sixth embodiment 6 differs from the fifth embodiment 5 in that electromagnetic radiation 70 that is transmitted through the waveguide interface 18 via a skinprint 30 is transmitted out of the waveguide 10 via an output grating coupler 17, as described previously in relation to the fourth embodiment 4.

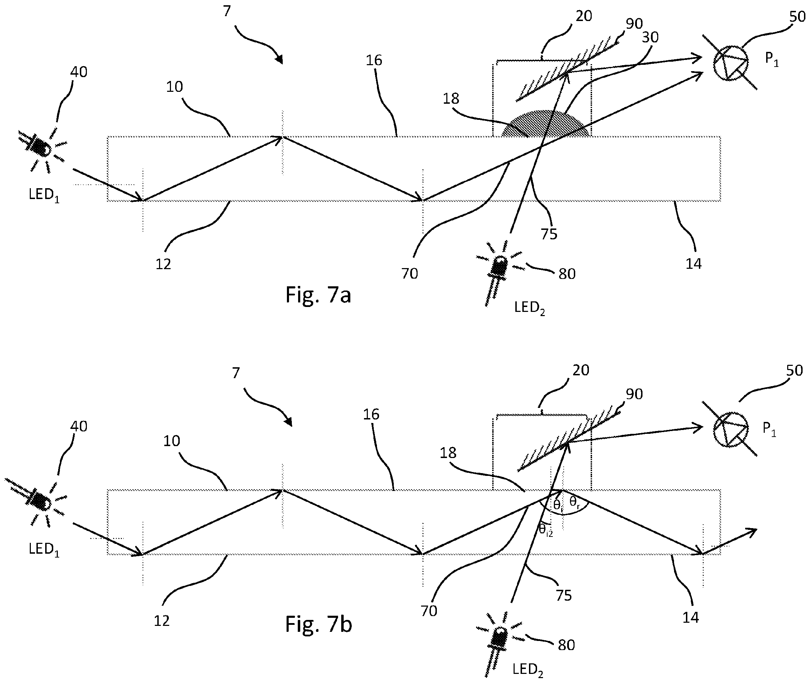

Figures 7a and 7b show a seventh embodiment 7 of the disclosure. The seventh embodiment 7 is similar to the first embodiment 1 and further comprises a secondary electromagnetic radiation source 80. The secondary electromagnetic radiation source 80 is located so that secondary radiation 75 emitted from the secondary electromagnetic radiation source 80 travels at an angle such that it transmits directly through the waveguide interface 18 whether or not a skinprint is present.

Accordingly, when no skinprint is present, only the secondary radiation 75 reaches the photodetector 50. This is because the primary electromagnetic radiation 70 from the primary electromagnetic radiation source 40 is reflected by the waveguide interface 18 rather than being transmitted through it. (A reflector 90 may be used to ensure that the secondary radiation, once out of the waveguide 10, is directed to the photodetector 50.)

When a skinprint 30 is present, primary radiation 70 from the primary radiation source 40 passes through the waveguide interface 18 such that both primary and secondary radiation 70, 75 reach the photodetector 50.

In one aspect of the seventh embodiment 7, one or both of the primary and secondary radiation sources 40, 80 may be pulsed. For example, if the primary radiation source 40 is constant and the secondary radiation source 80 is pulsed then the primary radiation 70 can be detected when the secondary radiation source 80 is off. A value for the secondary radiation 80 can be calculated by subtracting the measured primary radiation 70 from the measured combination of primary and secondary radiation when the secondary radiation source 80 is on.

If the primary and secondary radiation sources 40, 80 are of the same specification (e.g. in terms of brightness and spectrum) then they will both be affected by the material properties of the translucent waveguide 10 in the same way. Accordingly, it is possible by this technique to eliminate variations that arise from the use of different waveguides. This may be particularly appropriate where the waveguide 10 is a consumable product that is replaced with each test performed.

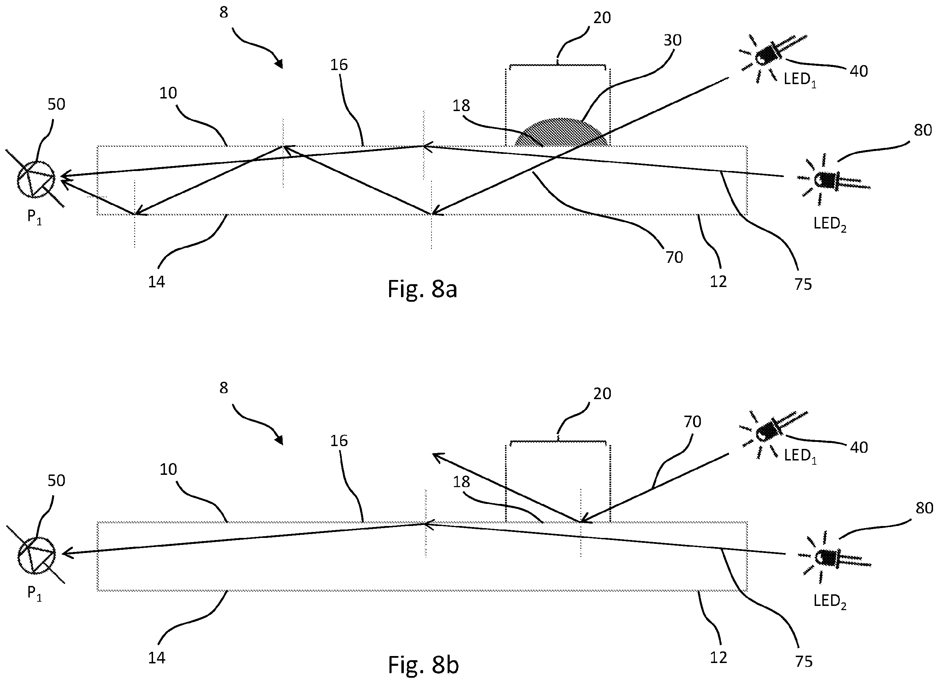

Figure 8a and 8b show an eighth embodiment of the disclosure. In common with the seventh embodiment 7, the eighth embodiment 8 comprises both primary and secondary electromagnetic radiation sources 40, 80. The primary and secondary electromagnetic radiation sources 40, 80 are both located towards a first end 12 of the translucent waveguide 10. The skinprint receiving region 20 is also located towards the first end 12 of the translucent waveguide 10. A photodetector 50 is located towards the second end 14 of the translucent waveguide 10.

In common with the fifth and sixth embodiments (and by contrast with the first, second, third, fourth and seventh embodiments), the direction of potential transmission through the waveguide interface 18 (in the presence of a skinprint) is into the translucent waveguide 10 rather than out of the translucent waveguide 10.

The primary electromagnetic radiation source 40 is located such that primary electromagnetic radiation 70 reaches the waveguide interface 18 from the exterior of the translucent waveguide 10 towards the first end 12 of the translucent waveguide 10. In addition, the fingerprint receiving region 20 is located on the first surface 16 of the translucent waveguide 10 also towards the first end 12 of the translucent waveguide 10. In the event that a skinprint is present, electromagnetic radiation 70 is transmitted through the waveguide interface 18 and into the translucent waveguide 10 for onward propagation towards the second end 14 of the translucent waveguide 14 through total internal reflection as shown schematically in Figure 8a. In the event that no skinprint is present, as shown in Figure 8b, primary electromagnetic radiation 70 from the primary electromagnetic radiation source 40 simply reflects off the waveguide interface 18 and does not enter the translucent waveguide 10.

The secondary electromagnetic radiation source 80 is located such that secondary radiation 75 is directed into the translucent waveguide 10 at an angle such that it propagates through the translucent waveguide 10 without opportunity for it to be coupled out of the translucent waveguide 10 until it reaches the second end 14 of the translucent waveguide in the region of the photodetector 50. This may be achieved by directing the secondary electromagnetic radiation 75 into the translucent waveguide 10 in a direction that is only marginally angled relative to the first surface 16 of the translucent waveguide 10 (or potentially substantially parallel to the first surface). In this way, the angle of travel of the secondary electromagnetic radiation 75 through the translucent waveguide 10 is such that neither the presence nor the absence of a skinprint 30 enables the radiation to be coupled out of the translucent waveguide 10, at least to any substantial degree.

Electromagnetic radiation (whether primary or secondary) that reaches the second end 14 of the translucent waveguide 10 is detected by the first photodiode 50. In the event that no skinprint 30 is present on the skinprint receiving region 20 (see Figure 8b), primary electromagnetic radiation 70 will not be coupled into the translucent waveguide 10 via the waveguide interface 18 and therefore only secondary radiation 75 will arrive at the photodiode 50. By contrast (see Figure 8a), in the event that a skinprint 30 is present on the skinprint receiving region 20, primary radiation 70 that is coupled into the translucent waveguide 10 via the waveguide interface 18 as a result of the presence of a skinprint 30, will arrive at the photodiode 50 in addition to secondary radiation 75.

As in the seventh embodiment, one or both of the primary and secondary radiation sources 40, 80 may be pulsed. For example, if the primary radiation source 40 is constant and the secondary radiation source 80 is pulsed then the primary radiation 70 can be detected in isolation when the secondary radiation source 80 is off. Where no skinprint 30 is present (such that no primary radiation would be expected to arrive at the photodiode 50) the photodiode would detect radiation only when the secondary radiation source 80 is on.

Alternatively, the secondary radiation source 80 may be constant and the primary radiation source 40 may be pulsed. In this way, where no skinprint is present there should be little difference between the radiation detected by the photodetector 50 regardless of the pulsed nature of the primary radiation 70 since the primary radiation 70 (when on) is not coupled into the translucent waveguide 10 and therefore does not reach the photodetector 50.

If the primary and secondary radiation sources 40, 80 are of the same specification (e.g. in terms of brightness and spectrum) then they will both be affected by the material properties of the translucent waveguide 10 in the same way. Accordingly, it is possible by this technique to eliminate variations that arise from the use of different waveguides. This may be particularly appropriate where the waveguide 10 is a consumable product that is replaced with each test performed.

In any embodiment involving both primary and secondary radiation, as an alternative to the pulsing strategy for separating primary and secondary radiation detected at the photodetector 50, it may be possible to use primary radiation having a different colour from that of the secondary radiation and use a colour sensitive photodetector to distinguish between the primary and secondary radiation. In short, any appropriate technique for distinguishing between primary and secondary radiation may be employed. Such techniques may include separation in the frequency domain, separation in the time domain, and separation in the colour domain. Whichever separation technique may be employed, the concept is to distinguish between primary radiation (main path) and secondary radiation (reference path).

The skilled person will appreciate that aspects of different embodiments described herein may be combined, including in ways not explicitly recited. For example, in the case of the seventh embodiment, it may be appropriate to use two photodiodes, in the manner of embodiments 2, 4, 5 and 6. Similarly, it may be appropriate to use a secondary electromagnetic radiation source in any of embodiments 1 to 6.

The skilled person will understand that refraction necessarily occurs when electromagnetic radiation passes between materials having different refractive indices (unless, of course, the difference of refractive indices is such as to result in total internal reflection). For the sake of clarity only, refraction is not shown in the schematic representations of Figures 1a to 8b.

The angle of incidence, θᵢ, at which the primary electromagnetic radiation 70 reaches the waveguide interface 18 is necessarily greater than the angle of incidence, θᵢ₂, at which the secondary electromagnetic radiation 75 reaches the waveguide interface 18. The exact values for θᵢ and θᵢ₂ will depend, among other things, on the refractive indices of the material used for the translucent waveguide 10 and the material (e.g. ambient air) on adjacent the waveguide interface 18 of the translucent waveguide 10.

Where total internal reflection of the primary electromagnetic radiation 70 having the angle of incidence, θᵢ, occurs at the waveguide interface 18 it reflects at an angle of reflection, θᵣ.

While the schematic Figures show the electromagnetic radiation taking only a single path, as the skilled person will readily appreciate, the path of the radiation will diverge. The single lines shown in the Figures are intended to represent the average path of the radiation and for clarity the divergence of radiation from the average path is not shown.

The primary and/or secondary electromagnetic radiation sources may be a source of visible spectrum radiation. The primary and/or secondary light source may be an LED, a filament bulb, a laser, a fluorescent bulb, or any other suitable source of electromagnetic radiation.

The primary and/or secondary electromagnetic radiation may be broad spectrum or narrow spectrum radiation. Potentially, it may be two non-contiguous ranges of narrow spectrum radiation. In some embodiments, the primary and secondary electromagnetic radiation may have the same properties (e.g. wavelength); in other embodiments the primary and secondary electromagnetic radiation may be selected to have different properties (e.g. wavelength).

While the specific embodiments employ one or more photodiodes as electromagnetic radiation detector(s), any appropriate electromagnetic radiation detector(s) may be used. Choice of electromagnetic radiation detector may be dependent, among other things, on the electromagnetic radiation source. Possible electromagnetic radiation detectors include: a photodiode; a phototransistor; a CCD sensor; and a light dependent resistor.

It may be appropriate to use a camera and/or a photomultiplier instead of or in addition to the electromagnetic radiation detector(s) shown in the specific embodiments. In particular, a camera may be used to provide an image of the electromagnetic radiation which may be compared to a database of such images for confirming the identity of a skinprint subject.

While the term skinprint is used throughout this specification, it will be appreciated that the most frequently used form of skinprint is currently the fingerprint (which includes the thumb-print). Nevertheless, other skinprints may be appropriate, such as (but not limited to) a hand-print, a toe-print, a footprint or an ear-print.

The translucent waveguide of any of the embodiments may be any translucent having appropriate properties of transmissivity of electromagnetic radiation of the appropriate wavelengths. The translucent waveguide may be transparent. It may be a glass slide or a plastic slide. An off the shelf slide may be particularly appropriate in embodiments where the translucent waveguide is intended to be a consumable item whereby a new translucent waveguide is employed for each test. If a plastic slide is employed, it may be produced by injection moulding and optionally it may be plasma treated to obtain desirable waveguide properties.

## Claims

1. A method of determining presence of residue of constituents of a skinprint (30) using an apparatus comprising: a primary electromagnetic radiation source (40); an electromagnetic radiation detector (50); and a translucent waveguide (10) comprising a first surface providing a waveguide interface (18) coincident with a skinprint receiving region (20);
the method comprising:
transmitting primary electromagnetic radiation (70) from the primary electromagnetic radiation source (40) towards the waveguide interface (18) at an angle of incidence relative to and on a first side of a normal line that is perpendicular to the waveguide interface (18), such that:
(a) where the waveguide interface (18) interfaces directly with ambient air, the primary electromagnetic radiation (70) incident on the waveguide interface (18) reflects in the waveguide interface (18) at an angle of reflection relative to and on a second side of the normal line opposite the first side; and
(b) where residue of constituents a skinprint (30) is present on the skinprint receiving region (20) such that the waveguide interface (18) interfaces with the residue of constituents of the skinprint (30) and the residue of constituents of the skinprint (30) interfaces with ambient air, at least a portion of the primary electromagnetic radiation (70) incident on the waveguide interface (18) is coupled into or out of the translucent waveguide (10) via the residue of constituents of the skinprint (30);
using the electromagnetic radiation detector (50) to determine an amount of primary electromagnetic radiation (70) transmitted through the waveguide interface (18) or reflected by the waveguide interface (18); and
where residue of constituents of the skinprint (30) is present, calculating a quality metric for the residue of constituents of the skinprint (30) based at least in part on an amount of primary electromagnetic radiation (70) detected by the electromagnetic radiation detector (50) to provide a value indicative of strength and/or extent of residue of constituents of the skinprint (30) on the skinprint receiving region (20).

2. The method of claim 1 wherein the portion of the primary electromagnetic radiation (70) that is transmitted through the waveguide interface (18) is transmitted in a direction such as to exit the translucent waveguide (10).

3. The method of claim 1 wherein the portion of the primary electromagnetic radiation (70) that is transmitted through the waveguide interface (18) is transmitted in a direction such as to enter the translucent waveguide (10).

4. The method of claim 3 wherein the step of transmitting primary electromagnetic radiation (70) from the primary electromagnetic radiation source (40) towards the waveguide interface (18) involves transmitting the primary electromagnetic radiation (70) at an angle such that the portion of primary electromagnetic radiation (70) that is transmitted through the waveguide interface (18) propagates through the translucent waveguide (10) by total internal reflection.

5. The method of any preceding claim wherein the step of using the electromagnetic radiation detector (50) to determine the amount of electromagnetic radiation transmitted through the waveguide interface (18) and/or reflected by the waveguide interface involves one of the following:
using the electromagnetic radiation detector (50) to detect an amount of electromagnetic radiation that exits the translucent waveguide (10) having passed through the waveguide interface (18); or
using the electromagnetic radiation detector (50) to detect an amount of electromagnetic radiation that exits the translucent waveguide (10) without having passed through the waveguide interface (18).

6. The method of any preceding claim wherein the step of calculating the quality metric comprises: comparing a signal from the electromagnetic radiation detector (50) to a reference value.

7. The method of claim 6 wherein the apparatus further comprises a secondary electromagnetic radiation source (80) configured to provide secondary electromagnetic radiation (75) and the method further comprises detecting the secondary electromagnetic radiation source (80) to provide the reference value.

8. The method of claim 7 and further wherein the step of calculating the quality comprises calculating a ratio of detected primary electromagnetic radiation (70) to detected secondary electromagnetic radiation (75).

9. The method of claim 7 or claim 8 further comprising pulsing either or both of the first electromagnetic radiation source (40) and the second electromagnetic radiation source (80).

10. An apparatus for determining presence of residue of constituents of a skinprint (30), the apparatus comprising: a primary electromagnetic radiation source (40); an electromagnetic radiation detector (50); and a translucent waveguide (10) comprising a first surface providing a waveguide interface (18) coincident with a skinprint receiving region (20);
wherein the apparatus is configured to:
transmit primary electromagnetic radiation (70) from the primary electromagnetic radiation source (40) towards the waveguide interface (18) at an angle of incidence relative to and on a first side of a normal line that is perpendicular to the waveguide interface (18), such that:
(a) where the waveguide interface (18) interfaces directly with ambient air, the primary electromagnetic radiation (70) incident on the waveguide interface (18) reflects in the waveguide interface (18) at an angle of reflection relative to and on a second side of the normal line opposite the first side; and
(b) where residue of constituents a skinprint (30) is present on the skinprint receiving region (20) such that the waveguide interface (18) interfaces with the residue of constituents of the skinprint (30) and the residue of constituents of the skinprint (30) interfaces with ambient air, at least a portion of the primary electromagnetic radiation (70) incident on the waveguide interface (18) is coupled into or out of the translucent waveguide (10) via the residue of constituents of the skinprint (30);
use the electromagnetic radiation detector (50) to determine an amount of primary electromagnetic radiation (70) transmitted through the waveguide interface (18) or reflected by the waveguide interface (18); and
where residue of constituents of the skinprint (30) is present, to calculate a quality metric for the residue of constituents of the skinprint (30) based at least in part on an amount of primary electromagnetic radiation (70) detected by the electromagnetic radiation detector (50) to provide a value indicative of strength and/or extent of residue of constituents of the skinprint (30) on the skinprint receiving region (20).

11. The apparatus of claim 10 further configured such that the portion of the primary electromagnetic radiation (70) that is transmitted through the waveguide interface (18) is transmitted in a direction such as to exit the translucent waveguide (10).

12. The apparatus of claim 10 further configured such that the portion of the primary electromagnetic radiation (70) that is transmitted through the waveguide interface (18) is transmitted in a direction such as to enter the translucent waveguide (10).

13. The apparatus of claim 12 further configured such that transmitting primary electromagnetic radiation (70) from the primary electromagnetic radiation source (40) towards the waveguide interface (18) involves transmitting the primary electromagnetic radiation (70) at an angle such that the portion of primary electromagnetic radiation (70) that is transmitted through the waveguide interface (18) propagates through the translucent waveguide (10) by total internal reflection.

14. The apparatus of claim any of claims 10 to 13 further configured such that use of the electromagnetic radiation detector (50) to determine the amount of electromagnetic radiation transmitted through the waveguide interface (18) and/or reflected by the waveguide interface involves one of the following:
using the electromagnetic radiation detector (50) to detect an amount of electromagnetic radiation that exits the translucent waveguide (10) having passed through the waveguide interface (18); or
using the electromagnetic radiation detector (50) to detect an amount of electromagnetic radiation that exits the translucent waveguide (10) without having passed through the waveguide interface (18).

15. The apparatus of claim any of claims 10 to 14 wherein the apparatus further comprises a secondary electromagnetic radiation source (80) configured to provide secondary electromagnetic radiation (75) and the apparatus is further configured to detect the secondary electromagnetic radiation source (80) to provide the reference value.

## Patentansprüche

1. Verfahren zur Bestimmung des Vorhandenseins von Rückständen von Bestandteilen eines Hautabdrucks (30) unter Verwendung einer Einrichtung, die Folgendes umfasst: eine primäre elektromagnetische Strahlungsquelle (40); einen elektromagnetischen Strahlungsdetektor (50); und einen lichtdurchlässigen Wellenleiter (10) mit einer ersten Oberfläche, die eine Wellenleitergrenzfläche (18) bereitstellt, die mit einem Hautabdruck-Empfangsbereich (20) zusammenfällt;
wobei das Verfahren Folgendes umfasst:
Senden primärer elektromagnetischer Strahlung (70) aus der primären elektromagnetischen Strahlungsquelle (40) in Richtung der Wellenleitergrenzfläche (18) unter einem Einfallswinkel relativ zu und auf einer ersten Seite einer Normalen, die senkrecht zu der Wellenleitergrenzfläche (18) steht, derart, dass:
(a) wenn die Wellenleitergrenzfläche (18) direkt an die Umgebungsluft angrenzt, die auf die Wellenleitergrenzfläche (18) einfallende primäre elektromagnetische Strahlung (70) an der Wellenleitergrenzfläche (18) unter einem Reflexionswinkel relativ zu und auf einer zweiten Seite der Normalen, die der ersten Seite gegenüberliegt, reflektiert wird; und
(b) wenn sich auf der Hautabdruck-Empfangsbereich (20) Rückstände von Bestandteilen eines Hautabdrucks (30) befinden, sodass die Wellenleitergrenzfläche (18) an die Rückstände von Bestandteilen des Hautabdrucks (30) angrenzt und die Rückstände von Bestandteilen des Hautabdrucks (30) an die Umgebungsluft angrenzen, mindestens ein Teil der auf die Wellenleitergrenzfläche (18) einfallenden primären elektromagnetischen Strahlung (70) über die Rückstände von Bestandteilen des Hautabdrucks (30) in den lichtdurchlässigen Wellenleiter (10) eingekoppelt oder aus diesem ausgekoppelt wird;
Verwenden des elektromagnetischen Strahlungsdetektors (50), um eine Menge an primärer elektromagnetischer Strahlung (70) zu bestimmen, die durch die Wellenleitergrenzfläche (18) hindurchtritt oder von der Wellenleitergrenzfläche (18) reflektiert wird; und
wenn Rückstände von Bestandteilen des Hautabdrucks (30) vorhanden sind, Berechnen einer Qualitätsmetrik für die Rückstände von Bestandteilen des Hautabdrucks (30) zumindest teilweise basierend auf einer Menge an primärer elektromagnetischer Strahlung (70), die von dem elektromagnetischen Strahlungsdetektor (50) erfasst wird, um einen Wert bereitzustellen, der eine Stärke und/oder ein Ausmaß von Rückständen von Bestandteilen des Hautabdrucks (30) auf dem Hautabdruck-Empfangsbereich (20) angibt.

2. Verfahren nach Anspruch 1, wobei der Anteil der primären elektromagnetischen Strahlung (70), der durch die Wellenleitergrenzfläche (18) hindurchtritt, in einer solchen Richtung gesendet wird, dass er aus dem lichtdurchlässigen Wellenleiter (10) austritt.

3. Verfahren nach Anspruch 1, wobei der Anteil der primären elektromagnetischen Strahlung (70), der durch die Wellenleitergrenzfläche (18) hindurchtritt, in einer solchen Richtung gesendet wird, dass er in den lichtdurchlässigen Wellenleiter (10) eintritt.

4. Verfahren nach Anspruch 3, wobei der Schritt des Sendens von primärer elektromagnetischer Strahlung (70) von der primären elektromagnetischen Strahlungsquelle (40) in Richtung der Wellenleitergrenzfläche (18) das Senden der primären elektromagnetischen Strahlung (70) unter einem solchen Winkel einbezieht, dass der Anteil der primären elektromagnetischen Strahlung (70), der durch die Wellenleitergrenzfläche (18) hindurchtritt, sich durch totale interne Reflexion in dem lichtdurchlässigen Wellenleiter (10) ausbreitet.

5. Verfahren nach einem vorstehenden Anspruch, wobei der Schritt des Verwendens des elektromagnetischen Strahlungsdetektor (50), um die Menge der durch die Wellenleitergrenzfläche (18) hindurchgetretenen und/oder von der Wellenleitergrenzfläche reflektierten elektromagnetischen Strahlung zu bestimmen, eines von Folgendem einbezieht:
Verwenden des elektromagnetischen Strahlungsdetektors (50), um eine Menge an elektromagnetischer Strahlung zu erfassen, die aus dem lichtdurchlässigen Wellenleiter (10) austritt, nachdem sie die Wellenleitergrenzfläche (18) passiert hat; oder
Verwenden des elektromagnetischen Strahlungsdetektors (50), um eine Menge an elektromagnetischer Strahlung zu erfassen, die aus dem lichtdurchlässigen Wellenleiter (10) austritt, ohne die Wellenleitergrenzfläche (18) passiert zu haben.

6. Verfahren nach einem vorstehenden Anspruch, wobei der Schritt des Berechnens der Qualitätsmetrik Folgendes umfasst: Vergleichen eines Signals aus dem elektromagnetischen Strahlungsdetektors (50) mit einem Referenzwert.

7. Verfahren nach Anspruch 6, wobei die Einrichtung weiter eine sekundäre elektromagnetische Strahlungsquelle (80) umfasst, die so konfiguriert ist, dass sie sekundäre elektromagnetische Strahlung (75) bereitstellt, und wobei das Verfahren weiter das Erfassen der sekundären elektromagnetischen Strahlungsquelle (80) umfasst, um den Referenzwert bereitzustellen.

8. Verfahren nach Anspruch 7 und weiter wobei der Schritt des Berechnens der Qualität ein Berechnen eines Verhältnisses von erfasster primärer elektromagnetischer Strahlung (70) zu erfasster sekundärer elektromagnetischer Strahlung (75) umfasst.

9. Verfahren nach Anspruch 7 oder Anspruch 8, weiter umfassend das pulsierende Betreiben einer oder beider von der ersten elektromagnetischen Strahlungsquelle (40) und der zweiten elektromagnetischen Strahlungsquelle (80).

10. Einrichtung zur Bestimmung des Vorhandenseins von Rückständen von Bestandteilen eines Hautabdrucks (30), wobei die Einrichtung Folgendes umfasst: eine primäre elektromagnetische Strahlungsquelle (40); einen elektromagnetischen Strahlungsdetektor (50); und einen lichtdurchlässigen Wellenleiter (10) mit einer ersten Oberfläche, die eine Wellenleitergrenzfläche (18) bereitstellt, die mit einem Hautabdruck-Empfangsbereich (20) zusammenfällt;
wobei die Einrichtung konfiguriert ist zum:
Senden primärer elektromagnetischer Strahlung (70) aus der primären elektromagnetischen Strahlungsquelle (40) in Richtung der Wellenleitergrenzfläche (18) unter einem Einfallswinkel relativ zu und auf einer ersten Seite einer Normalen, die senkrecht zu der Wellenleitergrenzfläche (18) steht, derart, dass:
(a) wenn die Wellenleitergrenzfläche (18) direkt an die Umgebungsluft angrenzt, die auf die Wellenleitergrenzfläche (18) einfallende primäre elektromagnetische Strahlung (70) an der Wellenleitergrenzfläche (18) unter einem Reflexionswinkel relativ zu und auf einer zweiten Seite der Normalen, die der ersten Seite gegenüberliegt, reflektiert wird; und
(b) wenn sich auf der Hautabdruck-Empfangsbereich (20) Rückstände von Bestandteilen eines Hautabdrucks (30) befinden, sodass die Wellenleitergrenzfläche (18) an die Rückstände von Bestandteilen des Hautabdrucks (30) angrenzt und die Rückstände von Bestandteilen des Hautabdrucks (30) an die Umgebungsluft angrenzen, mindestens ein Teil der auf die Wellenleitergrenzfläche (18) einfallenden primären elektromagnetischen Strahlung (70) über die Rückstände von Bestandteilen des Hautabdrucks (30) in den lichtdurchlässigen Wellenleiter (10) eingekoppelt oder aus diesem ausgekoppelt wird;
Verwenden des elektromagnetischen Strahlungsdetektors (50), um eine Menge an primärer elektromagnetischer Strahlung (70) zu bestimmen, die durch die Wellenleitergrenzfläche (18) hindurchtritt oder von der Wellenleitergrenzfläche (18) reflektiert wird; und
wenn Rückstände von Bestandteilen des Hautabdrucks (30) vorhanden sind, Berechnen einer Qualitätsmetrik für die Rückstände von Bestandteilen des Hautabdrucks (30) zumindest teilweise basierend auf einer Menge an primärer elektromagnetischer Strahlung (70), die von dem elektromagnetischen Strahlungsdetektor (50) erfasst wird, um einen Wert bereitzustellen, der eine Stärke und/oder ein Ausmaß von Rückständen von Bestandteilen des Hautabdrucks (30) auf dem Hautabdruck-Empfangsbereich (20) angibt.

11. Einrichtung nach Anspruch 10, die weiter so konfiguriert ist, dass der Anteil der primären elektromagnetischen Strahlung (70), der durch die Wellenleitergrenzfläche (18) hindurchtritt, in einer solchen Richtung gesendet wird, dass er aus dem lichtdurchlässigen Wellenleiter (10) austritt.

12. Einrichtung nach Anspruch 10, die weiter so konfiguriert ist, dass der Anteil der primären elektromagnetischen Strahlung (70), der durch die Wellenleitergrenzfläche (18) hindurchtritt, in einer solchen Richtung gesendet wird, dass er in den lichtdurchlässigen Wellenleiter (10) eintritt.

13. Einrichtung nach Anspruch 12, die weiter so konfiguriert ist, dass das Senden von primärer elektromagnetischer Strahlung (70) von der primären elektromagnetischen Strahlungsquelle (40) in Richtung der Wellenleitergrenzfläche (18) das Senden der primären elektromagnetischen Strahlung (70) unter einem solchen Winkel einbezieht, dass der Anteil der primären elektromagnetischen Strahlung (70), der durch die Wellenleitergrenzfläche (18) hindurchtritt, sich durch totale interne Reflexion in dem lichtdurchlässigen Wellenleiter (10) ausbreitet.

14. Einrichtung nach einem der Ansprüche 10 bis 13, die weiter so konfiguriert ist, dass das Verwenden des elektromagnetischen Strahlungsdetektor (50), um die Menge der durch die Wellenleitergrenzfläche (18) hindurchgetretenen und/oder von der Wellenleitergrenzfläche reflektierten elektromagnetischen Strahlung zu bestimmen, eines von Folgendem einbezieht:
Verwenden des elektromagnetischen Strahlungsdetektors (50), um eine Menge an elektromagnetischer Strahlung zu erfassen, die aus dem lichtdurchlässigen Wellenleiter (10) austritt, nachdem sie die Wellenleitergrenzfläche (18) passiert hat; oder
Verwenden des elektromagnetischen Strahlungsdetektors (50), um eine Menge an elektromagnetischer Strahlung zu erfassen, die aus dem lichtdurchlässigen Wellenleiter (10) austritt, ohne die Wellenleitergrenzfläche (18) passiert zu haben.

15. Einrichtung nach einem der Ansprüche 10 bis 14, wobei die Einrichtung weiter eine sekundäre elektromagnetische Strahlungsquelle (80) umfasst, die so konfiguriert ist, dass sie sekundäre elektromagnetische Strahlung (75) bereitstellt, und wobei die Einrichtung weiter so konfiguriert ist, dass sie die sekundäre elektromagnetische Strahlungsquelle (80) erfasst, um den Referenzwert bereitzustellen.

## Revendications

1. Procédé de détermination de la présence de résidus de constituants d'une empreinte cutanée (30) utilisant un appareil comprenant : une source de rayonnement électromagnétique primaire (40) ; un détecteur de rayonnement électromagnétique (50) ; et un guide d'ondes translucide (10) comprenant une première surface fournissant une interface de guide d'ondes (18) coïncidant avec une région de réception d'empreinte cutanée (20) ;
le procédé comprenant :
la transmission du rayonnement électromagnétique primaire (70) de la source de rayonnement électromagnétique primaire (40) vers l'interface de guide d'ondes (18) à un angle d'incidence par rapport à et sur un premier côté d'une ligne normale qui est perpendiculaire à l'interface de guide d'ondes (18), de sorte que :
(a) à l'endroit où l'interface de guide d'ondes (18) s'interface directement avec l'air ambiant, le rayonnement électromagnétique primaire (70) incident sur l'interface de guide d'ondes (18) se réfléchit dans l'interface de guide d'ondes (18) à un angle de réflexion par rapport à et sur un second côté de la ligne normale opposé au premier côté ; et
(b) à l'endroit où des résidus de constituants d'une empreinte cutanée (30) sont présents sur la région de réception d'empreinte cutanée (20) de sorte que l'interface de guide d'ondes (18) s'interface avec les résidus de constituants de l'empreinte cutanée (30) et que les résidus de constituants de l'empreinte cutanée (30) s'interfacent avec l'air ambiant, au moins une partie du rayonnement électromagnétique primaire (70) incident sur l'interface de guide d'ondes (18) est couplée dans ou hors du guide d'ondes translucide (10) à travers les résidus de constituants de l'empreinte cutanée (30) ;
l'utilisation du détecteur de rayonnement électromagnétique (50) pour déterminer une quantité de rayonnement électromagnétique primaire (70) transmise à travers l'interface de guide d'ondes (18) ou réfléchie par l'interface de guide d'ondes (18) ; et
à l'endroit où des résidus de constituants de l'empreinte cutanée (30) sont présents, le calcul d'une métrique de qualité pour les résidus de constituants de l'empreinte cutanée (30) sur la base au moins en partie d'une quantité de rayonnement électromagnétique primaire (70) détectée par le détecteur de rayonnement électromagnétique (50) afin de fournir une valeur indicative de l'intensité et/ou de l'étendue des résidus de constituants de l'empreinte cutanée (30) sur la région de réception d'empreinte cutanée (20).

2. Procédé selon la revendication 1, dans lequel la partie du rayonnement électromagnétique primaire (70) qui est transmise à travers l'interface de guide d'ondes (18) est transmise dans une direction telle qu'elle sorte du guide d'ondes translucide (10).

3. Procédé selon la revendication 1, dans lequel la partie du rayonnement électromagnétique primaire (70) qui est transmise à travers l'interface de guide d'ondes (18) est transmise dans une direction telle qu'elle entre dans le guide d'ondes translucide (10).

4. Procédé selon la revendication 3, dans lequel l'étape de transmission du rayonnement électromagnétique primaire (70) de la source de rayonnement électromagnétique primaire (40) vers l'interface de guide d'ondes (18) implique la transmission du rayonnement électromagnétique primaire (70) à un angle tel que la partie du rayonnement électromagnétique primaire (70) qui est transmise à travers l'interface de guide d'ondes (18) se propage à travers le guide d'ondes translucide (10) par réflexion interne totale.

5. Procédé selon une quelconque revendication précédente, dans lequel l'étape d'utilisation du détecteur de rayonnement électromagnétique (50) pour déterminer la quantité de rayonnement électromagnétique transmise à travers l'interface de guide d'ondes (18) et/ou réfléchie par l'interface de guide d'ondes implique l'une des opérations suivantes :
l'utilisation du détecteur de rayonnement électromagnétique (50) pour détecter une quantité de rayonnement électromagnétique qui sort du guide d'ondes translucide (10) après avoir traversé l'interface de guide d'ondes (18) ; ou
l'utilisation du détecteur de rayonnement électromagnétique (50) pour détecter une quantité de rayonnement électromagnétique qui sort du guide d'ondes translucide (10) sans avoir traversé l'interface de guide d'ondes (18).

6. Procédé selon une quelconque revendication précédente, dans lequel l'étape de calcul de la métrique de qualité comprend : la comparaison d'un signal provenant du détecteur de rayonnement électromagnétique (50) à une valeur de référence.

7. Procédé selon la revendication 6, dans lequel l'appareil comprend en outre une source de rayonnement électromagnétique secondaire (80) configurée pour fournir un rayonnement électromagnétique secondaire (75) et le procédé comprend en outre la détection de la source de rayonnement électromagnétique secondaire (80) pour fournir la valeur de référence.

8. Procédé selon la revendication 7, en outre dans lequel l'étape de calcul de la qualité comprend le calcul d'un rapport entre le rayonnement électromagnétique primaire détecté (70) et le rayonnement électromagnétique secondaire détecté (75).

9. Procédé selon la revendication 7 ou la revendication 8, comprenant en outre le fonctionnement par impulsions de la première source de rayonnement électromagnétique (40) ou de la seconde source de rayonnement électromagnétique (80), ou des deux.

10. Appareil pour déterminer la présence de résidus de constituants d'une empreinte cutanée (30), l'appareil comprenant : une source de rayonnement électromagnétique primaire (40) ; un détecteur de rayonnement électromagnétique (50) ; et un guide d'ondes translucide (10) comprenant une première surface fournissant une interface de guide d'ondes (18) coïncidant avec une région de réception d'empreinte cutanée (20) ;
dans lequel l'appareil est configuré pour :
transmettre le rayonnement électromagnétique primaire (70) de la source de rayonnement électromagnétique primaire (40) vers l'interface de guide d'ondes (18) à un angle d'incidence par rapport à et sur un premier côté d'une ligne normale qui est perpendiculaire à l'interface de guide d'ondes (18), de sorte que :
(a) à l'endroit où l'interface de guide d'ondes (18) s'interface directement avec l'air ambiant, le rayonnement électromagnétique primaire (70) incident sur l'interface de guide d'ondes (18) se réfléchit dans l'interface de guide d'ondes (18) à un angle de réflexion par rapport à et sur un second côté de la ligne normale opposé au premier côté ; et
(b) à l'endroit où des résidus de constituants d'une empreinte cutanée (30) sont présents sur la région de réception d'empreinte cutanée (20) de sorte que l'interface de guide d'ondes (18) s'interface avec les résidus de constituants de l'empreinte cutanée (30) et que les résidus de constituants de l'empreinte cutanée (30) s'interfacent avec l'air ambiant, au moins une partie du rayonnement électromagnétique primaire (70) incident sur l'interface de guide d'ondes (18) est couplée dans ou hors du guide d'ondes translucide (10) à travers les résidus de constituants de l'empreinte cutanée (30) ;
utiliser le détecteur de rayonnement électromagnétique (50) pour déterminer une quantité de rayonnement électromagnétique primaire (70) transmise à travers l'interface de guide d'ondes (18) ou réfléchie par l'interface de guide d'ondes (18) ; et
à l'endroit où des résidus de constituants de l'empreinte cutanée (30) sont présents, calculer une métrique de qualité pour les résidus de constituants de l'empreinte cutanée (30) sur la base au moins en partie d'une quantité de rayonnement électromagnétique primaire (70) détectée par le détecteur de rayonnement électromagnétique (50) afin de fournir une valeur indicative de l'intensité et/ou de l'étendue des résidus de constituants de l'empreinte cutanée (30) sur la région de réception d'empreinte cutanée (20).

11. Appareil selon la revendication 10, configuré en outre de sorte que la partie du rayonnement électromagnétique primaire (70) qui est transmise à travers l'interface de guide d'ondes (18) soit transmise dans une direction telle qu'elle sorte du guide d'ondes translucide (10).

12. Appareil selon la revendication 10, configuré en outre de sorte que la partie du rayonnement électromagnétique primaire (70) qui est transmise à travers l'interface de guide d'ondes (18) soit transmise dans une direction telle qu'elle entre dans le guide d'ondes translucide (10).

13. Appareil selon la revendication 12, configuré en outre de sorte que la transmission du rayonnement électromagnétique primaire (70) de la source de rayonnement électromagnétique primaire (40) vers l'interface de guide d'ondes (18) implique la transmission du rayonnement électromagnétique primaire (70) à un angle tel que la partie du rayonnement électromagnétique primaire (70) qui est transmise à travers l'interface de guide d'ondes (18) se propage à travers le guide d'ondes translucide (10) par réflexion interne totale.

14. Appareil selon l'une quelconque des revendications 10 à 13, configuré en outre de sorte que l'utilisation du détecteur de rayonnement électromagnétique (50) pour déterminer la quantité de rayonnement électromagnétique transmise à travers l'interface de guide d'ondes (18) et/ou réfléchie par l'interface de guide d'ondes implique l'une des opérations suivantes :
l'utilisation du détecteur de rayonnement électromagnétique (50) pour détecter une quantité de rayonnement électromagnétique qui sort du guide d'ondes translucide (10) après avoir traversé l'interface de guide d'ondes (18) ; ou
l'utilisation du détecteur de rayonnement électromagnétique (50) pour détecter une quantité de rayonnement électromagnétique qui sort du guide d'ondes translucide (10) sans avoir traversé l'interface de guide d'ondes (18).

15. Appareil selon l'une quelconque des revendications 10 à 14, dans lequel l'appareil comprend en outre une source de rayonnement électromagnétique secondaire (80) configurée pour fournir un rayonnement électromagnétique secondaire (75) et l'appareil est en outre configuré pour détecter la source de rayonnement électromagnétique secondaire (80) pour fournir la valeur de référence.
